# EUROPEAN PATENT SPECIFICATION

(11) **EP 1 984 017 B1**
(45) Date of publication and mention of the grant of the patent: **27.07.2016**
(21) Application number: 07716710.4
(22) Date of filing: 17.01.2007
(51) Int. Cl.: A61K 38/32, A61K 38/16, A61K 38/22, A61K 38/17

(54) **METHODS OF TREATING OR PREVENTING TISSUE DAMAGE CAUSED BY INCREASED BLOOD FLOW**
VERFAHREN ZUR THERAPIE ODER PROPHYLAXE VON DURCH ERHÖHTE DURCHBLUTUNG VERURSACHTEN GEWEBESCHÄDEN
PROCEDES DE TRAITEMENT OU DE PREVENTION D ENDOMMAGEMENT DE TISSUS CAUSES PAR UNE AUGMENTATION DU DEBIT SANGUIN

(30) Priority: 17.01.2006 US 759051 P
(43) Date of publication of application: 29.10.2008
(73) Proprietor: RegeneRx Biopharmaceuticals, Inc., Rockville, MD 20850 (US)
(72) Inventor: GOLDSTEIN, Allan, L., Washington, District Of Columbia 20037 (US); FINKELSTEIN, J.J., Chevy Chase, Maryland 20815 (US)
(74) Representative: Klunker . Schmitt-Nilson . Hirsch
(86) International application number: PCT/US2007/001206
(87) International publication number: WO 2007/084544

(56) References cited:
- WO-A-2005/087805
- US-A- 5 871 437
- US-A1- 2004 220 111
- US-A1- 2005 027 184
- BOCK-MARQUETTE ILDIKO ET AL: "Thymosin beta4 activates integrin-linked kinase and promotes cardiac cell migration, survival and cardiac repair" NATURE, NATURE PUBLISHING GROUP, LONDON, UK, vol. 432, no. 7016, 25 November 2004 (2004-11-25), pages 466-472, XP002358431 ISSN: 0028-0836
- GOLDSTEIN ET AL: "Thymosin beta4: actin-sequestering protein moonlights to repair injured tissues" TRENDS IN MOLECULAR MEDICINE, ELSEVIER CURRENT TRENDS, vol. 11, no. 9, 1 September 2005 (2005-09-01), pages 421-429, XP005065335 ISSN: 1471-4914

## Description

### BACKGROUND OF THE INVENTION

### 1. Field of the Invention

The present invention relates to the field of preventing tissue damage caused by an increase in blood flow.

### 2. Description of the Background Art

There are a number of drugs, devices and medical procedures which are utilized to unclog or increase blood flow through arteries and other blood vessels. However, unclogging of blood vessels sometimes permits a large amount of blood, containing oxygen, free radicals and other chemicals, to rush into a tissue site with a potential for causing damage to the tissue.

There remains a need in the art for methods and compositions for preventing tissue damage caused by an increase in blood flow.

WO 2005/087805 A1 discloses a method of treatment for treating, preventing, inhibiting or reducing extracellular matrix build-up in a body tissue or a bodily fluid transport vessel, in a subject, the method including administering to a subject in need of such treatment an effective amount of a composition including a peptide agent including amino acid sequence LKKTET, a conservative variant thereof, or a peptide agent that stimulates production of an LKKTET peptide or a conservative variant thereof, in the tissue.

### SUMMARY OF THE INVENTION

Subject-matter of the present invention is a polypeptide for use in a method for preventing tissue damage occurring subsequent to affecting an increase in blood flow through a blood vessel which is in communication with said tissue, and a pharmaceutical combination as claimed in the independent claims. Embodiments of the invention are claimed in the respective dependent claims.

In accordance with the use according to the present invention, a method of preventing tissue damage occurring subsequent to affecting an increase in blood flow in a blood vessel which is in communication with said tissue, comprises administering an effective amount of a composition comprising a tissue damage-preventing polypeptide selected from the group consisting of Thymosin beta 4 (Tß4), an isoform of Tß4, Tß4 sulfoxide, an LKKTET peptide, an LKKTNT peptide, and an actin polymerization-modulating peptide. The composition is administered to said tissue during at least one of before, during or after affecting said increase in blood flow.

### DETAILED DESCRIPTION OF THE INVENTION

The present invention is based on a discovery that peptides such as thymosin β4 (Tβ4) and other e.g. actin-sequestering peptides or peptide fragments which may contain amino acid sequence LKKTET or LKKTNT or conservative variants thereof (hereinafter sometimes referred to as a "tissue damage-preventing or -reducing peptide(s)"), promote healing or prevention of cardiac, neuro or other tissue damage and other changes associated with an increase in blood flow. Such tissue damage-preventing peptides comprise at least one of Thymosin beta 4 (Tß4), an isoform of Tß4, an N-terminal fragment of Tß4, a C-terminal fragment of Tß4, Tß4 sulfoxide, an LKKTET peptide, an LKKTNT peptide, an actin-sequestering peptide, an actin binding peptide, an actin-mobilizing peptide, an actin polymerization-modulating peptide, or a conservative variant thereof. Included are N- or C-terminal fragments or variants, which may or may not include KLKKTET and LKKTETQ. Tß4 has been suggested as being a factor in angiogenesis in rodent models. However, there heretofore has been no known indication that such properties may be useful in treating tissue damage caused by an increase in blood flow. Without being bound to any particular theory, these peptides may have the capacity to promote repair, healing and prevention by having the ability to induce terminal deoxynucleotidyl transferase (a non-template directed DNA polymerase), to decrease and modulate the levels of one or more inflammatory cytokines or chemokines, and to act as a chemotactic and/or angiogenic factor for cells and thus heal and prevent tissue damage caused by an increase in blood flow.
Therefore, subject-matter of the present invention, and claimed herein, are polypeptides selected from the group consisting of thymosin beta 4 (Tβ4), an isoform of Tβ4, Tβ4 sulfoxide, an LKKTET peptide, an LKKTNT peptide and an actin polymerization-modulating peptide, for use in a method for preventing tissue damage occurring subsequent to affecting an increase in blood flow through a blood vessel which is in communication with said tissue.

The invention is particularly useful in conjunction with use of agents (e.g., drugs, devices or procedures) utilized to unclog or increase blood flow through arteries and other blood vessels. In order to prevent tissue damage occurring subsequent to affecting an increase in blood flow through a blood vessel which is in communication with the tissue, the tissue damage-preventing peptide can be administered before, during and/or after affecting the increase in blood flow.

Agents which may be utilized to affect an increase in blood flow through a blood vessel include, but are not limited to, aspirin, tPA, streptokinase, plasminogen, anti-clotting agents, anistreplase, reteplase, tenecteplase and/ or heparin. The tissue damage-preventing peptide can be administered before, during and/or after blood flow is increased in conjunction therewith. Amounts of such agents which are effective in increasing blood flow through blood vessels are included within the range of 0.001-1,000 mg. The invention also is applicable to compositions comprising such blood flow-increasing agents and a tissue damage-preventing peptide.

Devices and procedures which may be utilized to affect an increase in blood flow through a blood vessel include, but are not limited to, arterial stents, venous stents, cardiac catheterizations, carotid stents, aortic stents, pulmonary stents, angioplasty, bypass surgery and/or neurosurgery. The tissue damage-preventing peptide can be administered before, during and/or after blood flow is increased in conjunction therewith.

Indications to which the invention may be applicable include, but are not limited to, trauma induced ischemia (neuro or cardio), disease induced ischemia, idiopathic ischemia and/or stroke. The tissue damage-preventing peptide can be administered before, during and/or after blood flow is increased in conjunction therewith.

Tissue damage-preventing peptides as described herein, can prevent and/or limit the apoptic death of brain and other neurovascular cells and tissues following ischemic, infectious, pathological, toxic or traumatic damage by upregulating metabolic and signaling enzymes such as the phosphatidylinositol 3-kinase (P13-K)/Akt (protein kinase β) pathway. Upregulating P13-K/ Akt and downstream phosphorylated Bad and proline rich Akt survival kinase protects neuronal cells during hypoxic insults. In addition, tissue damage-preventing peptides as described herein, by virtue of their ability to downregulate inflammatory cytokines such as IL-18 and chemokines such as IL-8 and enzymes such as caspace 2, 3, 8 and 9 protect neuronal cells and facilitate healing of nervous tissue.

Tissue damage-preventing peptides as described herein, when administered immediately before, during and/or after administration of a thrombolytic agent as described herein will limit neuronal damage due to a hypoxic insult by inducing neuronal tissue to undergo a form of hibernation characterized by modulation of the P13-K/ Akt signaling pathways and decreased neuronal apoptosis, and decreased inflammatory chemokine, cytokine and capase activity.

Tissue damage-preventing peptides as described herein, prevent neurotoxicity in the brain and spinal cord following ischemic or traumatic injury by preventing glutamate induced neurotoxicity. Uncontrolled release of glutamate, an excitatory neurotransmitter, from damaged brain and nervous tissues is a primary mediator of mitochondrial dysfunction and energy mechanisms in the cell which results in several inflammatory reactions, mechanical stress altered trophic signals and death of affected nervous cells and tissues.

As noted above, the tissue damage-preventing peptide may be administered before, during and/or after affecting an increase in blood flow through a blood vessel which is in communication with the tissue. Delivery pathways include, but are not limited to, parenteral, oral, nasal, pulmonary, intracardiac, intravenous, transdermal and/or liposomal.

Thymosin β4 was initially identified as a protein that is up-regulated during endothelial cell migration and differentiation in vitro. Thymosin β4 was originally isolated from the thymus and is a 43 amino acid, 4.9 kDa ubiquitous polypeptide identified in a variety of tissues. Several roles have been ascribed to this protein including a role in a endothelial cell differentiation and migration, T cell differentiation, actin sequestration and vascularization.

Described herein is a method of treatment for promoting healing and prevention of damage and inflammation associated with tissue damage caused by an increase in blood flow comprising administering to a subject in need of such treatment an effective amount of a composition comprising a tissue damage-reducing peptide comprising amino acid sequence LKKTET or LKKTNT, or a conservative variant thereof having a tissue damage-reducing activity, preferably Thymosin β4, an isoform of Thymosin β4, or an antagonist of Thymosin β4. The method may also utilize oxidized Tβ4.

Compositions described herein include Thymosin β4 (Tβ4), T β4 isoforms, oxidized T β4, polypeptides or any other actin sequestering or bundling proteins having actin binding domains, or peptide fragments which may or may not comprise or consist essentially of the amino acid sequence LKKTET or LKKTNT or conservative variants thereof, having tissue damage-reducing activity. International Application Serial No. PCT/US99/17282, discloses isoforms of Tβ4 which may be useful in accordance with the present invention as well as amino acid sequence LKKTET and conservative variants thereof. International Application Serial No. PCT/GB99/00833 (WO 99/49883), discloses oxidized Thymosin β4 which may be utilized in accordance with the present invention. Although the present invention is described primarily hereinafter with respect to Tβ4 and Tβ4 isoforms, it is to be understood that the following description is intended to be equally applicable to amino acid sequence LKKTET, LKKTNT, LKKTETQ, or KLKKTET peptides and fragments comprising or consisting essentially of LKKTET, or LKKTNT or LKKTETQ or KLKKTET, as well as oxidized Thymosin β4 and other tissue damage-preventing peptides.

Described herein is a method for healing and preventing inflammation and damage in a subject by contacting the tissue site with an effective amount of a tissue damage-reducing composition which contains Tβ4 or a Tβ4 isoform or other tissue damage-preventing or -reducing peptides as described herein. The contacting may be direct or systemically. Examples of contacting the damaged site include contacting the site with a composition comprising the tissue damage-preventing or -reducing peptide alone, or in combination with at least one agent that enhances penetration, or delays or slows release of tissue damage-preventing or -reducing peptides into the area to be treated. The administration may be directly or systemically. Examples of administration include, for example, direct application, injection or infusion, with a solution, lotion, salve, gel cream, paste spray, suspension, dispersion, hydrogel, ointment, foam, oil or solid comprising a tissue damage-preventing or -reducing peptide as described herein. Administration may include, for example, intravenous, intraperitoneal, intramuscular or subcutaneous injections, or inhalation, transdermal or oral administration of a composition containing the tissue damage-preventing or - reducing peptide, etc. This applies equally to the polypeptide and the pharmaceutical combination claimed herein. A subject may be a mammal, preferably human.

The tissue damage-preventing peptide may be administered in any suitable tissue damage preventing amount. For example, tissue damage-preventing peptide may be administered in dosages within the range of about 0.0001-1,000,000 micrograms, more preferably about 0.01-5,000 micrograms, still more preferably about 0.1-50 micrograms, most preferably in amounts within the range of about 1-30 micrograms.

A composition in accordance with the present invention can be administered daily, every other day, etc., with a single administration or multiple administrations per day of administration, such as applications 2, 3, 4 or more times per day of administration.

Tβ4 isoforms have been identified and have about 70%, or about 75%, or about 80% or more homology to the known amino acid sequence of Tβ4. Such isoforms include, for example, Tβ4ala, Tβ9, Tβ10, Tβ11, Tβ12, Tβ13, Tβ14 and Tβ15. Similar to Tβ4, the Tβ10 and Tβ15 isoforms have been shown to sequester actin. Tβ4, Tβ10 and Tβ15, as well as these other isoforms share an amino acid sequence, LKKTET or LKKTNT, that appears to be involved in mediating actin sequestration or binding. Although not wishing to be bound to any particular theory, the activity of Tβ4 isoforms may be due, in part, to the ability to regulate the polymerization of actin. β-thymosins appear to depolymerize F-actin by sequestering free G-actin. Tβ4's ability to modulate actin polymerization may therefore be due to all, or in part, its ability to bind to or sequester actin via the LKKTET or LKKTNT sequence. Thus, as with Tβ4, other tissue damage-preventing or -reducing proteins which may bind or sequester actin, or modulate actin polymerization, including Tβ4 isoforms having the amino acid sequence LKKTET or LKKTNT, are likely to be effective, alone or in a combination with Tβ4, as set forth herein. Specific peptides indicated below are used in embodiments of the invention.

Thus, it is specifically contemplated that known Tβ4 isoforms, such as Tβ4ala, Tβ9, Tβ10, Tβ11, Tβ12, Tβ13, Tβ14 and Tβ15, as well as Tβ4 isoforms not yet identified, will be useful in the invention. As such Tβ4 isoforms are useful in the invention, including for the use in methods practiced in a subject. The invention therefore further provides pharmaceutical compositions comprising Tβ4, as well as Tβ4 isoforms Tβ4ala, Tβ9, Tβ10, Tβ11, Tβ12, Tβ13, Tβ14 and Tβ15, and a pharmaceutically acceptable carrier.

In addition, other proteins that can modulate actin polymerization, as demonstrated in an appropriate polymerization assay, or identified by the presence of an amino acid sequence LKKTET or LKKTNT can similarly be employed in the invention. Such proteins include gelsolin, vitamin D binding protein (DBP), profilin, cofilin, adsevertin, propomyosin, fincilin, depactin, DnaseI, vilin, fragmin, severin, capping protein, β-actinin and acumentin. As the polypeptides are used in methods including those practiced in a subject, the invention further provides pharmaceutical compositions comprising gelsolin, vitamin D binding protein (DBP), profilin, cofilin, depactin, DnaseI, vilin, fragmin, severin, capping protein, β-actinin and acumentin as set forth herein. Thus, the invention includes the use of a tissue damage-preventing polypeptide which may comprise the amino acid sequence LKKTET or LKKTNT (which may be within its primary amino acid sequence).

As used herein, the term "conservative variant" or grammatical variations thereof denotes the replacement of an amino acid residue by another, biologically similar residue. Examples of conservative variations include the replacement of a hydrophobic residue such as isoleucine, valine, leucine or methionine for another, the replacement of a polar residue for another, such as the substitution of arginine for lysine, glutamic for aspartic acids, or glutamine for asparagine, and the like.

Tβ4 has been localized to a number of tissue and cell types and thus, agents which stimulate the production of Tβ4 or another tissue damage-preventing or-reducing peptide can be added to a composition of the disclosure to effect tissue damage-preventing or -reducing peptide production from a tissue and/or a cell. Such agents include members of the family of growth factors, such as insulin-like growth factor (IGF-1), platelet derived growth factor (PDGF), epidermal growth factor (EGF), transforming growth factor beta (TGF-β), basic fibroblast growth factor (bFGF), thymosin α1 (Tα1) and vascular endothelial growth factor (VEGF). More preferably, the agent is transforming growth factor beta (TGF-β) or other members of the TGF-β superfamily. Compositions disclosed herein may reduce tissue damage caused by an increase in blood flow by effectuating growth of the connective tissue through extracellular matrix deposition, cellular migration and vascularization.

In accordance with one embodiment, subjects are treated, in addition to treatment with the tissue damage-preventing polypeptide, with an agent that stimulates production in the subject of a tissue damage-preventing or -reducing peptide as defined herein.

Additionally, agents that assist or stimulate healing of tissue damage caused by an increase in blood flow event may be added to a composition along with tissue damage-preventing peptide. Such agents include angiogenic agents, growth factors, agents that direct differentiation of cells. For example, and not by way of limitation, tissue damage-preventing peptides alone or in combination can be added in combination with any one or more of the following agents: VEGF, KGF, FGF, PDGF, TGFβ, IGF-1, IGF-2, IL-1, prothymosin α and thymosin α1 in an effective amount.

The invention also includes a pharmaceutical composition comprising a therapeutically effective amount of tissue damage-preventing peptide in a pharmaceutically acceptable carrier. Such carriers include, *inter alia,* those listed herein.

The actual dosage, formulation or composition that heals or prevents inflammation, damage and degeneration associated with tissue damage caused by an increase in blood flow may depend on many factors, including the size and health of a subject. However, persons of ordinary skill in the art can use teachings describing the methods and techniques for determining clinical dosages as disclosed in PCT/US99/17282, supra, and the references cited therein, to determine the appropriate dosage to use in the present invention.

Suitable formulations include tissue damage-preventing peptide at a concentration within the range of about 0.001 - 50% by weight, more preferably within the range of about 0.01 - 0.1% by weight, most preferably about 0.05% by weight.

The therapeutic approaches described herein involve various routes of administration or delivery of reagents or compositions comprising the tissue damage-preventing compounds of the invention, including any conventional administration techniques to a subject. The methods described and compositions using or containing tissue damage-preventing compounds of the invention may be formulated into pharmaceutical compositions by admixture with pharmaceutically acceptable non-toxic excipients or carriers.

Described herein is also the use of antibodies which interact with tissue damage-preventing or -reducing peptides or functional fragments thereof. Antibodies which consists essentially of pooled monoclonal antibodies with different epitopic specificities, as well as distinct monoclonal antibody preparations are provided. Monoclonal antibodies are made from antigen containing fragments of the protein by methods well known to those skilled in the art as disclosed in PCT/US99/17282, supra. The term antibody as used herein is meant to include monoclonal and polyclonal antibodies.

Further described herein is a method of treating a subject by administering an effective amount of an agent which modulates tissue damage-preventing or - reducing peptide gene expression. The term "modulate" refers to inhibition or suppression of tissue damage-preventing or -reducing peptide expression when tissue damage-preventing or -reducing peptide is over expressed, and induction of expression when tissue damage-preventing or -reducing peptide is under expressed. The term "effective amount" means that amount of modulating agent which is effective in modulating tissue damage-preventing or -reducing peptide gene expression resulting in effective treatment. An agent which modulates Tβ4 or tissue damage-preventing or -reducing peptide gene expression may be a polynucleotide for example. The polynucleotide may be an antisense, a triplex agent, or a ribozyme. For example, an antisense directed to the structural gene region or to the promoter region of Tβ4 may be utilized.

Additionally described herein is a method for utilizing compounds that modulate Tβ4 or tissue damage-preventing or -reducing peptide activity. Compounds that affect Tβ4 or tissue damage-preventing or -reducing peptide activity (e.g., antagonists and agonists) include peptides, peptidomimetics, polypeptides, chemical compounds, minerals such as zincs, and biological agents.

While not be bound to any particular theory, healing or prevention of inflammation or damage associated with tissue damage caused by an increase in blood flow may be promoted by inducing terminal deoxynucleotidyl transferase (a non-template directed DNA polymerase), to decrease the levels of one or more inflammatory cytokines, or chemokines, and to act as a chemotactic factor for endothelial cells, and thereby promoting healing or preventing tissue damage caused by an increase in blood flow or other degenerative or environmental factors. The following examples illustrate effects of Tβ4.

### Example 1

Synthetic Tβ4 and an antibody to Tβ4 was provided by RegeneRx Biopharmaceuticals, Inc. (3 Bethesda Metro Center, Suite 700, Bethesda, MD 20814) and were tested in a collagen gel assay to determine their effects on the Transformation of cardiac endothelial cells to mesenchymal cells. It is well established that development of heart valves and other cardiac tissue are formed by epithelial-mesenchymal transformation and that defects in this process can cause serious cardiovascular malformation and injury during development and throughout life. At physiological concentrations Tβ4 markedly enhances the transformation of endocardial cells to mesenchymal cells in the collagen gel assay. Furthermore, an antibody to Tβ4 inhibited and blocked this transformation. Transformation of atrioventricular endocardium into invasive mesenchyme is an aspect of the formation and maintenance of normal cardiac tissue and in the formation of heart valves.

### Example 2

Regulatory pathways involved in cardiac development may have utility in reprogramming cardiomycytes to aid in cardiac repair. In studies of genes expressed during cardiac morphogenesis, it was found that the forty-three amino acid peptide thymosin β4 was expressed in the developing heart. Thymosin β4 has numerous functions with the most prominent involving sequestration of G-actin monomers and subsequent effects on actin-cytoskeletal organization necessary for cell motility, organogenesis and other cell biological events. Recent domain analyses indicate that β4-thymosins can affect actin assembly based on their carboxy-terminal affinity for actin. In addition to cell motility, thymosin β4 may affect transcriptional events by influencing Rho-dependent gene expression or chromatin remodeling events regulated by nuclear actin.

Here, it is shown that thymosin β4 can stimulate migration of cardiomyocytes and endothelial cells and promote survival of cardiomyocytes. The LIM domain protein PINCH and Integrin Linked Kinase (ILK), both of which are necessary for cell migration and survival, formed a complex with thymosin β4 that resulted in phosphorylation of the survival kinase Akt/PKB. Inhibition of Akt phosphorylation reversed thymosin β4's effects on cardiac cells. Treatment of adult mice with thymosin β4 after coronary ligation resulted in increased phosphorylation of Akt in the heart, enhanced early myocyte survival within twenty-four hours and improved cardiac function. These results indicate that an endogenous protein expressed during cardiogenesis may be re-deployed to protect myocardium in the setting of acute coronary events.

### Results

### Developmental Expression of Thymosin β4

Expression of thymosin β4 in the developing brain was previously reported, as was expression in the cardiovascular system, although not in significant detail. Whole mount RNA in situ hybridization of embryonic day (E) 10.5 mouse embryos revealed thymosin β4 expression in the left ventricle, outer curvature of the right ventricle and cardiac outflow tract. Radioactive in situ hybridization indicated that thymosin β4 transcripts were enriched in the region of cardiac valve precursors known as endocardial cushions. Cells in this region are derived from endothelial cells that undergo mesenchymal transformation, migrate away from the endocardium and invade a swelling of extracellular matrix separating the myocardium and endocardium. In addition to endocardial cells, a subset of myocardial cells migrate and populate the cushion region and this process is necessary for septation and remodeling of the cardiac chambers. Using immunohistochemistry, it was found that thymosin β4-expressing cells in the cushions also expressed cardiac muscle actin, suggesting that thymosin β4 was present in migratory cardiomyocytes that invade the endocardial cushion. Finally, thymosin β4 transcripts and protein were also expressed at E9.5-E11.5 in the ventricular septum and the less differentiated, more proliferative region of the myocardium, known as the compact layer, which migrates into the trabecular region as the cells mature. Outflow tract myocardium that migrates from the anterior heart field also expressed high levels of thymosin β4 protein.

### Secreted Thymosin β4 Stimulated Cardiac Cell Migration and Survival

Although thymosin β4 is found in the cytosol and nucleus and functions intracellularly, we found that conditioned medium of Cos 1 cells transfected with myctagged thymosin β4 contained thymosin β4 detectable by Western blot, consistent with previous reports of thymosin β4 secretion and presence in wound fluid. Upon expression of thymosin β4 on the surface of phage particles added extracellularly to embryonic cardiac explants, it was found that an anti-phage antibody coated the cell surface and was ultimately detected intracellularly in the cytosol and nucleus while control phage was not detectable. Similar observations were made using biotinylated thymosin β4. These data indicated that secreted thymosin β4 may be internalized into cells, although the mechanism of cellular entry remains to be determined.

To test the effects of secreted thymosin β4 on cardiac cell migration, an embryonic heart explant system designed to assay cell migration and transformation events on a three-dimensional collagen gel was utilized. In this assay, explants of adjacent embryonic myocardium and endocardium from valve-forming regions were placed on a collagen gel with the endocardium adjacent to the collagen. Signals from cardiomyocytes induce endocardial cell migration but myocardial cells do not normally migrate onto the collagen in significant numbers. In contrast, upon addition of thymosin β4 to the primary explants, it was observed that a large number of spontaneously beating, cardiac muscle actin-positive cells had migrated away from the explant. No significant difference in cell death or proliferative rate based on TUNEL assay or phosho-histone H3 immunostaining, respectively, was observed in these cells compared to control cells.

To test the response of post-natal cardiomyocytes, primary rat neonatal cardiomyoctyes were cultured on laminin-coated glass and treated the cells with phosphate buffered saline (PBS) or thymosin β4. Similar to embryonic cardiomyocytes, it was found that the migrational distance of thymosin β4-treated neonatal cardiomyocytes was significantly increased compared to control (p<.05). In addition to thymosin β4's effects on myocardial cell migration, a similar effect was observed on endothelial migration in the embryonic heart explant assay. Exposure of E11.5 explants to thymosin β4 resulted in an increased number of migrating endothelial cells, compared to PBS (p<.01).

Primary culture of neonatal cardiomyocytes typically survived for approximately one to two weeks with some cells beating up to two weeks when grown on laminin-coated slides in our laboratory. Surprisingly, neonatal cardiomyocytes survived significantly longer upon exposure to thymosin β4 with rhythmically contracting myocytes visible for up to 28 days. In addition, the rate of beating was consistently faster in thymosin β4-treated neonatal cardiomyocytes (95 vs. 50 beats per minute, p<.02), indicating either a change in cell-cell communication or more vigorous cardiomyocytes.

### Thymosin β4 Activates ILK and Ak/Protein Kinase B

To investigate the potential mechanisms through which thymosin β4 might be influencing cell migration and survival events, thymosin β4 interacting proteins were searched. The amino-terminus of thymosin β4 was fused with affi-gel beads resulting in exposure of the carboxy-terminus that allowed identification of previously unknown interacting proteins but prohibited association with actin. An E9.5-12.5 mouse heart T7 phage cDNA library was synthesized and screened by phage display and thymosin (34-interacting clones were enriched and confirmed by ELISA. PINCH, a LIM domain protein, was most consistently isolated in this screen and interacted with thymosin β4 in the absence of actin (ELISA). PINCH and integrin linked kinase (ILK) interact directly with one another and indirectly with the actin cytoskeleton as part of a larger complex involved in cell-extracellular matrix interactions known as the focal adhesion complex. PINCH and ILK are required for cell motility and for cell survival, in part by promoting phosphorylation of the serine-threonine kinase Akt/ protein kinase B, a central kinase in survival and growth signaling pathways. Plasmids encoding thymosin β4 were transfected with or without PINCH or ILK in cultured cells and it was found that thymosin β4 co-precipitated with PINCH or ILK independently. Moreover, PINCH, ILK and thymosin β4 consistently immunoprecipitated in a common complex, although the interaction of ILK with thymosin β4 was weaker than with PINCH. The PINCH interaction with thymosin β4 mapped to the fourth and fifth LIM domains of PINCH while the amino terminal ankryin domain of ILK was sufficient for thymosin β4 interaction.

Because recruitment of ILK to the focal adhesion complex is important for its activation, the effects of thymosin β4 on ILK localization and expression were assayed. ILK detection by immunocytochemistry was markedly enhanced around the cell edges after treatment of embryonic heart explants or C2C12 myoblasts with synthetic thymosin β4 protein (10ng/100ul) or thymosin β4-expressing plasmid. Western analysis indicated a modest increase in ILK protein levels in C2C12 cells, suggesting that the enhanced immunofluoresence may be in part due to altered localization by thymosin β4. It was found that upon thymosin β4 treatment of C2C 12 cells, ILK was functionally activated, evidenced by increased phosphorylation of its known substrate Akt, using a phospho-specific antibody to serine 473 of Akt, while total Akt protein was unchanged. The similar effects of extracellularly administered thymosin β4 and transfected thymosin β4 were consistent with previous observations of internalization of the peptide and suggested an intracellular rather than an extracellular role in signaling for thymosin β4. Because thymosin β4 sequesters the pool of G-actin monomers, the effects on ILK activation were dependent on thymosin β4's role in regulating the balance between polymerized F-actin and monomeric G-actin were tested. F-actin polymerization was inhibited using C3 transferase and also F-actin formation was promoted with an activated Rho, but neither intervention affected the ILK activation observed after treatment of COS1 or C2C 12 cells with thymosin β4.

To determine if activation of ILK was necessary for the observed effects of thymosin β4, a well-described ILK inhibitor, wortmannin, was employed, which inhibits ILK's upstream kinase, phosphatidylinositol 3-kinase (PI3-kinase). Using myocardial cell migration and beating frequency as assays for thymosin β4 activity, embryonic heart explants were cultured as described above in the presence of thymosin β4 with or without wortmannin. Consistent with ILK mediating thymosin β4's effects, a significant reduction in myocardial cell migration and beating frequency was observed upon inhibition of ILK (p<.05). Together, these results supported a physiologically significant interaction of thymosin β4-PINCH-ILK within the cell and suggested that this complex may mediate some of the observed effects of thymosin β4 relatively independent of actin polymerization.

### Thymosin β4 Promotes Cell Survival after Myocardial Infarction and Improves Cardiac Function

Because of thymosin β4's effects on survival and migration of cardiomyocytes cultured in vitro and phosphorylation of Akt, it was tested whether thymosin β4 might aid in cardiac repair in vivo after myocardial damage. Myocardial infarctions in fifty-eight adult mice were created by coronary artery ligation and treated half with systemic, intracardiac, or systemic plus intracardiac thymosin β4 immediately after ligation and the other half with PBS. Intracardiac injections were done with collagen (control) or collagen mixed with thymosin β4. All forty-five mice that survived two weeks later were interrogated for cardiac function by random-blind ultrasonagraphy at 2 and 4 weeks after infarction by multiple measurements of cardiac contraction. Four weeks after infarction, left ventricles of control mice had a mean fractional shortening of 23.2 +/-1.2% (n=22, 95% confidence interval); in contrast, mice treated with thymosin β4 had a mean fractional shortening of 37.2 +/-1.8 % (n=23, 95% confidence intervals; p<.0001). As a second measure of ventricular function, two-dimensional echocardiographic measurements revealed that the mean fraction of blood ejected from the left ventricle (ejection fraction) in thymosin β4 treated mice was 57.7 +/- 3.2 % (n=23, 95% confidence interval; p<.0001) compared to a mean of 28.2 +/- 2.5 % (n=22, 95% confidence interval) in control mice after coronary ligation. The greater than 60% or 100% improvement in cardiac fractional shortening or ejection fraction, respectively, suggested a significant improvement with exposure to thymosin β4, although cardiac function remained depressed compared to sham operated animals (∼60% fractional shortening; ∼75% ejection fraction). Finally, the end diastolic dimensions (EDD) and end systolic dimensions (ESD) were significantly higher in the control group, indicating that thymosin β4 treatment resulted in decreased cardiac dilation after infarction, consistent with improved function. Remarkably, the degree of improvement when thymosin β4 was administered systemically through intraperitoneal injections or only locally within the cardiac infarct was not statistically different, suggesting that the beneficial effects of thymosin β4 likely occurred through a direct effect on cardiac cells rather than through an extracardiac source. Control cardiac injections were performed with the same collagen vehicle making it unlikely that an endogenous reaction to the injection contributed to the cardiac recovery.

To determine the manner in which thymosin β4 improved cardiac function, multiple serial histologic sections of hearts treated with or without thymosin β4 were examined. Trichrome stain at three levels of section revealed that the size of scar was reduced in all mice treated with thymosin β4 but was not different between systemic or local delivery of thymosin β4, consistent with the echocardiographic data above. Quantification of scar volume using six levels of sections through the left ventricle of a subset of mice demonstrated significant reduction of scar volume in thymosin β4 treated mice (p<.05). We did not detect significant cardiomyocyte proliferation or death at three, six, eleven or fourteen days after coronary ligation in PBS or thymosin β4 treated hearts. However, twenty-four hours after ligation we found a striking decrease in cell death by TUNEL assay (green) in thymosin β4 treated cardiomyocytes, confirmed by double-labeling with muscle-actin antibody (red). TUNEL positive cells that were also myocytes were rare in the thymosin β4 group but abundant in the control hearts. Consistent with this observation, it was found that the left ventricle fractional shortening three days after infarction was 39.2 +/- 2.34% (n=4, 95% confidence interval) with intracardiac thymosin β4 treatment compared to 28.8 +/-2.26% (n=4, 95% confidence interval) in controls (p<.02); ejection fraction was 64.2 +/-6.69% or 44.7 +/-8.4%, respectively (p<.02), suggesting early protection by thymosin β4. Finally, there was no detection of any differences in the number of c-kit, Sca-1 or Abcg2 positive cardiomyocytes between treated and untreated hearts and the cell volume of cardiomyocytes in thymosin β4 treated animals was similar to mature myocytes, suggesting that the thymosin β4-induced improvement was unlikely to be influenced by recruitment of known stem cells into the cardiac lineage. Thus, the decreased scar volume and preserved function of thymosin β4 treated mice were likely due to early preservation of myocardium after infarction through thymosin β4's effects on survival of cardiomyocytes.

Because thymosin β4 upregulates ILK activity and Akt phosphorylation in cultured cells, the effects on these kinases in vivo were tested. By western blot it was found that the level of ILK protein was increased in heart lysates of mice treated with thymosin β4 after coronary ligation compared with PBS treated mice. Correspondingly, phospho-specific antibodies to Akt-5473 revealed an elevation in the amount of phosphorylated Akt-5473 in mice treated with thymosin β4, consistent with the effects of thymosin β4 on ILK described earlier. Total Akt protein was not increased. These observations in vivo were consistent with the effects of thymosin β4 on cell migration and survival demonstrated in vitro and suggest that activation of ILK and subsequent stimulation of Akt may in part explain the enhanced cardiomyocyte survival induced by thymosin β4, although it is unlikely that a single mechanism is responsible for the full repertoire of thymosin β4's cellular effects.

### Discussion

The evidence presented here suggests that thymosin β4, a protein involved in cell migration and survival during cardiac morphogenesis, may be re-deployed to minimize cardiomyocyte loss after cardiac infarction. Given the roles of PINCH, ILK and Akt, the data is consistent with this complex playing a central role in thymosin β4's effects on cell motility, survival and cardiac repair. Thymosin β4's ability to prevent cell death within twenty four hours after coronary ligation likely leads to the decreased scar volume and improved ventricular function observed in mice. Although thymosin β4 activation of ILK is likely to have many cellular effects, the activation of Akt may be the dominant mechanism through which thymosin β4 promotes cell survival. This is consistent with Akt's proposed effect on cardiac repair when over-expressed in mouse marrow-derived stem cells administered after cardiac injury, although this likely occurs in a non-cell autonomous fashion.

The early effect of thymosin β4 in protecting the heart from cell death was reminiscent of myocytes that are able to survive hypoxic insult by "hibernating". While the mechanisms underlying hibernating myocardium are unclear, alterations in metabolism and energy usage appear to promote survival of cells. Induction agents such as thymosin β4 may alter cellular properties in a manner similar to hibernating myocardium, possibly allowing time for endothelial cell migration and new blood vessel formation.

Here, we show that the G-actin sequestering peptide thymosin β4 promotes myocardial and endothelial cell migration in the embryonic heart and retains this property in post-natal cardiomyocytes. Survival of embryonic and postnatal cardiomyocytes in culture was also enhanced by thymosin β4. It was found that thymosin β4 formed a functional complex with PINCH and Integrin Linked Kinase (ILK), resulting in activation of the survival kinase Akt/PKB, which was necessary for thymosin β4's effects on cardiomyocytes. After coronary artery ligation in mice, thymosin β4 treatment resulted in upregulation of ILK and Akt activity in the heart, enhanced early myocyte survival and improved cardiac function. These findings indicate that thymosin β4 promotes cardiomyocyte migration, survival and repair and is a novel therapeutic target in the setting of acute myocardial damage.

### Methods

### RNA in situ hybridization

Whole-mount or section RNA in situ hybridization of E 9.5-12.5 mouse embryos was performed with digoxigenin-labeled or S-labelled antisense riboprobes synthesized from the 3'UTR region of mouse thymosin β4 cDNA that did not share homology with the closely related transcript of thymosin β10.

### Immunohistochemistry

Embryonic or adult cardiac tissue was embedded in paraffin and sections used for immunohistochemistry. Embryonic heart sections were incubated with anti-thymosin β4 that does not recognize thymosin β10. Adult hearts were sectioned at ten equivalent levels from the base of the heart to the apex. Serial sections were used for trichrome sections and reaction with sarcomeric a-actinin, c-kit, Sca-1, Abcg2, and BrdU antibodies and for TUNEL assay (Intergen Company # S7111).

### Collagen gel migration assay

Outflow tract was dissected from E11.5 wild type mouse embryos and placed on collagen matrices as previously described. After 10 hours of attachment explants were incubated in 30ng/300µl thymosin β4 in PBS, PBS alone or thymosin β4 and 100nM wortmannin. Cultures were carried out for 3-9 days at 37°C 5% CO₂ and fixed in 4% paraformaldehyde in PBS for 10 min at RT. Cells were counted for quantification of migration and distance using at least three separate explants under each condition for endothelial migration and eight separate explants for myocardial migration.

### Immunocytochemistry on collagen gel explants

Paraformaldehyde-fixed explants were permeabilized for 10 min at RT with Permeabilize solution (10mM PIPES pH6.8; 50mMNaC1; 0.5% Triton X-100; 300mM Sucrose; 3mM MgCl₂) and rinsed with PBS 2 x 5 min at RT. After a series of blocking and rinsing steps, detection antibodies were used and explants rinsed and incubated with Equilibration buffer (Anti-Fade kit) 10 min at room temperature. Explants were scooped to a glass microscope slide, covered, and examined by fluorescein microscopy. TUNEL assay was performed using ApopTag Plus Fluorescein In Situ Apoptosis detection kit (Intergen Company # S7111) as recommended.

### Embryonic T7 phage display cDNA library

Equal amounts of mRNA were isolated and purified from E 9.5-12.5 mouse embryonic hearts by using Straight A's mRNA Isolation System (Novagen, Madison WI). cDNA was synthesized by using T7Select10-3 OrientExpress cDNA Random Primer Cloning System (Novagen, Madison WI). The vector T7Select10-3 was employed to display random primed cDNA at the C-terminus of 5-15 phage 10B coat protein molecules. Expression of the second coat protein 10A was induced. After EcoRl and Hind III digestion, inserts were ligated into T7 select10-3 vector (T7 select System Manual, Novagen). The vector was packaged and complexity of the library was 10⁷. Packaged phage was amplified in a log phase 0.5 L culture of BLT5615 E. *Coli* strain at 37 °C for 4 h. The cell debris was removed by centrifugation and the phage was precipitated with 8% polyethylene glycol. Phage was extracted from the pellet with 1M NaCl/lOmM Tris-HCl pH 8.0/1mM EDTA and purified by CsCI gradient ultracentrifugation. Purified phages were dialyzed against PBS and stored in 10% glycerol at -80 °C.

### T7 phage biopanning

300 ul of Affi-Gel 15 (Bio-Rad Laboratories) was coupled with 12 ug of synthesized thymosin β4 protein (RegeneRx) following the manufacturers manual, likely via amino terminal lysine residues. After blocking with 3% BSA in PBS for 1 h the gel was transferred to a column and washed with 10 ml of PBS, 2ml of 1 % SDS/PBS and 1 ml of PBS/0.05% Tween-20 (PBST) x 4.10⁹ pfu's of the T7 phage embryonic heart library (100x of the complexity) in 500ul of PBST was applied to the column and incubated for 5 min to achieve low stringency biopanning. Unbound phages were washed with 50ml of PBS. Bound phages were eluted in 2.0 ml of 1 % SDS. 10 µl of eluted phages was titered and the rest of the phages were immediately amplified in 0.5 L of log phase BLT5615 E. *Coli* culture until lysis. Cell debris was removed by centrifugation, lysate was titered and 10⁹ pfu's of phages were used for the next round of biopanning. 4 rounds of biopanning were performed and 30 single colonies were picked after the 2^{nd} 3^{rd} and 4^{th} round before amplification, respectively for sequence analysis. Single colonies containing greater than ten amino acids were amplified and used for ELISA confirmation assay.

### ELISA confirmation assay

MaxiSorp Nunc-Immuno Plates (Nalgene Nunc International) were coated with 1µg/100 µl of synthesized thymosin β4 peptide overnight then washed with PBS and blocked with 3 % BSA. 10⁹ pfu's of amplified single phage colonies were added in PBST to each well separately and incubated for 1.5 h at RT. T7 wild type phage was used as negative control. Unbound phages were removed by washing with PBS (x4), and bound phages were eluted by adding 200 µl of 1% SDS/PBS to the wells for 1 h at RT.

### Coimmunoprecipitation

Cos and 10T1/2 cells were transfected with thymosin β4, PINCH and/or ILK and lysates precipitated with antibodies to each as previously described. Western blots were performed using anti-ILK polyclonal antibody (Santa Cruz), anti-thymosin β4 polyclonal antibody and anti-myc or anti-FLAG antibody against tagged versions of PINCH.

### Animals and surgical procedures

Myocardial infarction was produced in fifty-eight male C57BL/6J mice at 16 weeks of age (25-30 g) by ligation of the left anterior descending coronary artery as previously described. Twenty-nine of the ligated mice received thymosin β4 treatment immediately following ligation and the remaining twenty-nine received PBS injections. Treatment was given intracardiac with thymosin β4 (200ng in lOul collagen) or with 10 ul of collagen; intraperitoneally with thymosin zβ (150µg in 300µl PBS) or with 3000 of PBS; or by both intracardiac and intraperitoneal injections. Intraperitoneal injections were given every three days until mice were sacrificed. Doses were based on previous studies of thymosin β4 biodistribution. Hearts were removed, weighed and fixed for histologic sectioning. Additional mice were operated on in a similar fashion for studies 0.5, 1, 3, 6 and 11 days after ligation.

### Analysis of cardiac function by echocardiography

Echocardiograms to assess systolic function were performed using M-mode and 2-dimensional measurements as described previously. The measurements represented the average of six selected cardiac cycles from at least two separate scans performed in random-blind fashion with papillary muscles used as a point of reference for consistency in level of scan. End diastole was defined as the maximal left ventricle (LV) diastolic dimension and end systole was defined as the peak of posterior wall motion. Single outliers in each group were omitted for statistical analysis. Fractional shortening (FS), a surrogate of systolic function, was calculated from LV dimensions as follows: FS = EDD - ESD / EDD x 100%. Ejection fraction (EF) was calculated from two-dimensional images. EDD, end diastolic dimension; ESD, end systolic dimension.

### Calculation of scar volume

Scar volume was calculated using six sections through the heart of each mouse using Openlab 3.03 software (Improvision) similar to previously described. Percent area of collagen deposition was measured on each section in blinded fashion and averaged for each mouse.

### Statistical analyses

Statistical calculations were performed using standard t-test of variables with 95% confidence intervals.

Thymosin β4 promotes myocardial and endothelial cell migration in the embryonic heart and retains this property in postnatal cardiomyocytes. Survival or embryonic and postnatal cardiomyocytes in culture was also enhanced by thymosin β4. Thymosin β4 forms a functional complex with PINCH and integrin-linked kinase (ILK), resulting in activation of the survival kinase Akt (also know as protein kinase B). After coronary artery ligation in mice, thymosin β4 treatment results in upregulation of ILK and Akt activity in the heart, enhances early myocyte survival and improves cardiac function. These findings indicate that thymosin β4 promotes cardiomyocyte migration, survival and repair and the pathway it regulates is a new therapeutic target in the setting of acute myocardial damage.

### Example 3

Synthetic Tβ4 and an antibody to Tβ4 was provided by RegeneRx Biopharmaceuticals, Inc. (3 Bethesda Metro Center, Suite 700, Bethesda, MD 20814) and were tested in a collagen gel assay to determine their effects on the Transformation of cardiac endothelial cells to mesenchymal cells. It is well established that development of heart valves and other cardiac tissue are formed by epithelial-mesenchymal transformation and that defects in this process can cause serious cardiovascular malformation and injury during development and throughout life. At physiological concentrations Tβ4 markedly enhances the transformation of endocardial cells to mesenchymal cells in the collagen gel assay. Furthermore, an antibody to Tβ4 inhibited and blocked this transformation. Transformation of atrioventricular endocardium into invasive mesenchyme is critical in the formation and maintenance of normal cardiac tissue and in the formation of heart valves.

### Example 4

0.1ug to 1ug per kg body weight of thymosin B4 (TB4) is administered by cardiac catheterization immediately following angioplasty and the patient then receives 600ug to 6mg TB4 intravenously per kg body weight (BW) two to four times per day for a period up to seven days. The amount and duration of treatment is dependent on the extent of ventricular damage following an acute myocardial infarction as measured by electrocardiography and nuclear imaging at the time of angiography and during the initial hospitalization of the patient.

### Example 5

0.1ug to 1ug per kg/BW of TB4 is administered by cardiac catherization immediately after angioplasty and/or stenting. The patient then receives by IV administration 600ug to 6mg/kg BW two to four times/day for a period of up to seven days following an MI. Preservation of heart muscle and reduction in restenosis is measured by electrocardiography and monitored by nuclear imaging or other diagnostic methods.

### Example 6

TB4 is administered IV at a dosage of 1mg to 10mg/kg BW/daily for up to 30 days to reduce coronary blockage due to plaque formation.

### Example 7

Thymosin beta 4, and other tissue damage-preventing or -reducing peptides as described herein are administered with drugs, devices and procedures utilized to unclog or increase blood flow through arties and other blood vessels, including aspirin, tPA, streptokinase, plasminogen, anti-clotting agents, anistreplase, reteplase, tenecteplase, heparin, arterial stents, venous stents, cardiac catheterizations, carotid stents, aortic stents, pulmonary stents, angioplasty, bypass surgery and/or neurosurgery. The tissue damage-reducing polypeptides are administered before, during and/or after the increase in blood flow brought about by the drugs, devices and procedures. The tissue damage-reducing polypeptides reduce and/or prevent tissue damage associated with increase in blood flow.

## Claims

1. A polypeptide selected from the group consisting of Thymosin beta 4 (Tβ4), an isoform of Tβ4, Tβ4 sulfoxide, an LKKTET peptide, an LKKTNT peptide, and an actin polymerization-modulating peptide, for use in a method for preventing tissue damage occurring subsequent to affecting an increase in blood flow through a blood vessel which is in communication with said tissue.

2. The polypeptide for the use of claim 1 wherein said polypeptide is selected from the group consisting of polypeptides comprising amino acid sequence KLKKTET or LKKTETQ, Thymosin β4 (Tβ4), an isoform of Tβ4 and oxidized Tβ4.

3. The polypeptide for the use of claim 1 wherein said polypeptide is selected from the group consisting of Tβ4, Tβ4ala, Tβ9, Tβ10, Tβ11, Tβ12, Tβ13, Tβ14, Tβ15, gelsolin, vitamin D binding protein (DBP), profilin, cofilin, adservertin, propomyosin, fincilin, depactin, Dnasel, vilin, fragmin, severin, capping protein, β-actinin or acumentin.

4. The polypeptide for the use of claim 1 wherein said polypeptide is Thymosin β4.

5. The polypeptide for the use of any one of claims 1 to 4, wherein said polypeptide is recombinant or synthetic.

6. The polypeptide for the use of any one of claims 1 to 5, wherein said increase in blood flow is affected by administration of at least one of aspirin, tPA, streptokinase, plasminogen, anti-clotting agents, anistreplase, reteplase, tenecteplase or heparin.

7. The polypeptide for the use of any one of claims 1 to 5, wherein said increase in blood flow is affected by at least one of arterial stents, venous stents, cardiac catheterizations, carotid stents, aortic stents, pulmonary stents, angioplasty, bypass surgery or neurosurgery.

8. The polypeptide for the use of any one of claims 1 to 7, wherein said tissue damage is neurological damage.

9. The polypeptide for the use of claim 8, wherein said damage is due to a treatment of trauma induced ischemia, disease induced ischemia, idiopathic ischemia or stroke.

10. A pharmaceutical combination comprising a tissue damage-preventing polypeptide as claimed in any one of claims 1 to 9 having tissue damage-preventing activity, the combination further comprising a blood flow increasing-effective amount of a blood flow-increasing agent wherein said polypeptide and said blood flow-increasing agent may be administered separately or together for the use as claimed in any one of claims 1 to 9.

11. The combination of claim 10 for the use as claimed in anyone of claims 1 to 9, wherein said blood flow-increasing agent comprises aspirin, tPA, streptokinase, plasminogen, anti-clotting agents, anistreplase, reteoplase, tenecteplase, or heparin.

12. The combination of claim 10 for the use as claimed in any one of claims 1 to 9, wherein said blood flow-increasing agent comprises tPA or streptokinase.

13. The combination of any one of claims 10 to 12 for the use as claimed in anyone of claims 1 to 9, wherein said polypeptide is for administration before, during, or after administration of said blood flow-increasing agent to increase blood flow.

## Patentansprüche

1. Polypeptid, das ausgewählt ist aus der Gruppe, die aus Thymosin beta 4 (Tβ4), einer Isoform von Tβ4, Tβ4-Sulfoxid, einem LKKTET-Peptid, einem LKKTNT-Peptid und einem die Actin-Polymerisierung modulierenden Peptid besteht, zur Verwendung in einem Verfahren zum Verhindern einer Gewebeschädigung, die nach dem Eintreten einer Erhöhung des Blutflusses durch ein Blutgefäß, das mit dem Gewebe in Verbindung ist, auftritt.

2. Polypeptid zur Verwendung nach Anspruch 1, wobei das Polypeptid ausgewählt ist aus der Gruppe, die aus Polypeptiden, die die Aminosäuresequenz KLKKTET oder LKKTETQ aufweisen, Thymosin β4 (Tβ4), einer Isoform von Tβ4 und oxidiertem Tβ4 besteht.

3. Polypeptid zur Verwendung nach Anspruch 1, wobei das Polypeptid ausgewählt ist aus der Gruppe, die aus Tβ4, Tβ4ala, Tβ9, Tβ10, Tβ11, Tβ12, Tβ13, Tβ14, Tβ15, Gelsolin, Vitamin D bindendem Protein (DBP), Profilin, Cofilin, Adsevertin, Propomyosin, Fincilin, Depactin, Dnasel, Vilin, Fragmin, Severin, Capping Protein, β-Actinin oderAcumentin besteht.

4. Polypeptid zur Verwendung nach Anspruch 1, wobei das Polypeptid Thymosin β4 ist.

5. Polypeptid zur Verwendung nach einem der Ansprüche 1 bis 4, wobei das Polypeptid rekombinant oder synthetisch ist.

6. Polypeptid zur Verwendung nach einem der Ansprüche 1 bis 5, wobei die Erhöhung des Blutflusses bewirkt wird durch Verabreichung mindestens eines der Mittel Aspirin, tPA, Streptokinase, Plasminogen, gerinnungshemmenden Mitteln, Anistreplase, Reteplase, Tenecteplase oder Heparin.

7. Polypeptid zur Verwendung nach einem der Ansprüche 1 bis 5, wobei die Erhöhung des Blutflusses bewirkt wird durch arterielle Stents und/oder Venenstents und/oder Herz-Katheterisierungen und/oder Karotis-Stents und/oder Aorten-Stents und/oder Lungen-Stents und/oder Angioplastie und/oder Bypass-Chirurgie und/oder Neurochirurgie.

8. Polypeptid zur Verwendung nach einem der Ansprüche 1 bis 7, wobei die Gewebeschädigung eine neurologische Schädigung ist.

9. Polypeptid zur Verwendung nach Anspruch 8, wobei die Schädigung bedingt ist durch eine Behandlung von Trauma-induzierter Ischämie, Krankheits-induzierter Ischämie, idiopathischer Ischämie oder Schlaganfall.

10. Pharmazeutische Kombination aufweisend ein Gewebeschädigung verhinderndes Polypeptid, wie es in einem der Ansprüche 1 bis 9 beansprucht ist, mit Gewebeschädigung verhindernder Aktivität, wobei die Kombination außerdem eine zur Erhöhung des Blutflusses wirksame Menge eines den Blutfluss erhöhenden Mittels aufweist, wobei das Polypeptid und das den Blutfluss erhöhende Mittel getrennt oder zusammen verabreicht werden können, zur Verwendung wie sie in einem derAnsprüche 1 bis 9 beansprucht ist.

11. Kombination nach Anspruch 10 für die Verwendung, wie sie in einem der Ansprüche 1 bis 9 beansprucht ist, wobei das den Blutfluss erhöhende Mittel Aspirin, tPA, Streptokinase, Plasminogen, gerinnungshemmende Mittel, Anistreplase, Reteplase, Tenecteplase oder Heparin aufweist.

12. Kombination nach Anspruch 10 für die Verwendung, wie sie in einem der Ansprüche 1 bis 9 beansprucht ist, wobei das den Blutfluss erhöhende Mittel tPA oder Streptokinase aufweist.

13. Kombination nach einem der Ansprüche 10 bis 12 für die Verwendung, wie sie in einem der Ansprüche 1 bis 9 beansprucht ist, wobei das Polypeptid für eine Verabreichung vor, während, oder nach der Verabreichung des den Blutfluss erhöhenden Mittels zur Erhöhung des Blutflusses ist.

## Revendications

1. Polypeptide sélectionné parmi le groupe consistant en de la thymosine bêta-4 (Tβ4), une isoforme de Tβ4, un sulfoxyde de Tβ4, un peptide LKKTET, un peptide LKKTNT, et un peptide de modulation de polymérisation d'actine, à utiliser dans un procédé destiné à empêcher l'endommagement de tissu se produisant suite à l'affectation d'une augmentation de flux sanguin à travers un vaisseau sanguin en communication avec ledit tissu.

2. Polypeptide destiné à l'utilisation selon la revendication 1, dans lequel ledit polypetide est sélectionné parmi le groupe consistant en des polypeptides comprenant une séquence d'acides aminées KLKKTET ou LKKTETQ, de la thymosine (Tβ4), une isoforme de Tβ4 et de la Tβ4 oxydée.

3. Polypeptide destiné à l'utilisation selon la revendication 1, dans lequel ledit polypeptide est sélectionné parmi le groupe consistant en Tβ4, Tβ4ala, Tβ9, Tβ10, Tβ11, Tβ12, Tβ13, Tβ14, Tβ15, de la gelsoline, une protéine de liaison de la vitamine D (DBP), de la profiline, de la cofiline, de la adsevertine, de la propomyosine, de la finciline, de la dépactine, du DNasel, de la viline, de la fragmine, de la séverine, une protéine de coiffage, de la ß-actinite ou de l'acumentine.

4. Polypeptide destiné à l'utilisation selon la revendication 1, dans lequel ledit polypeptide est de la thymosine β4.

5. Polypeptide destiné à l'utilisation selon l'une quelconque des revendications 1 à 4, dans lequel ledit polypeptide est recombinant ou synthétique.

6. Polypeptide destiné à l'utilisation selon l'une quelconque des revendications 1 à 5, dans lequel ladite augmentation de flux sanguin est affectée par l'administration d'au moins un élément parmi : de l'aspirine, du tPA, de la streptokinase, un plasminogène, des agents anti-coagulants, de l'anistreplase, du retéplase, du ténectéplase ou de l'héparine.

7. Polypeptide destiné à l'utilisation selon l'une quelconque des revendications 1 à 5, dans lequel ladite augmentation de flux sanguin est affectée par au moins un élément parmi des stents artériels, des stents veineux, des cathétérismes cardiaques, des stents carotidiens, des stents aortiques, des stents pulmonaires, l'angioplastie, la chirurgie de pontage, la neurochirurgie.

8. Polypeptide destiné à l'utilisation selon l'une quelconque des revendications 1 à 7, dans lequel ledit endommagement de tissu est un endommagement neurologique.

9. Polypeptide destiné à l'utilisation selon la revendication 8, dans lequel ledit endommagement est dû à un traitement d'une ischémie d'origine traumatique, une ischémie issue d'une maladie, une ischémie idiopathique ou un accident vasculaire cérébral.

10. Combinaison pharmaceutique comprenant un polypeptide empêchant l'endommagement de tissu selon l'une quelconque des revendications 1 à 9, présentant une activité visant à empêcher l'endommagement de tissu, la combinaison comprenant en outre une quantité effective pour l'augmentation de flux sanguin d'un agent d'augmentation de flux sanguin, dans laquelle ledit polypeptide et ledit agent d'augmentation de flux sanguin peuvent être administrés séparément ou ensemble pour l'utilisation selon l'une quelconque des revendications 1 à 9.

11. Combinaison selon la revendication 10 pour l'utilisation selon l'une quelconque des revendications 1 à 9, dans laquelle ledit agent d'augmentation de flux sanguin comprend : de l'aspirine, du tPA, de la streptokinase, un plasminogène, des agents anti-coagulants, de l'anistreplase, du retéplase, du ténectéplase, ou de l'héparine.

12. Combinaison selon la revendication 10 pour l'utilisation selon l'une quelconque des revendications 1 à 9, dans laquelle ledit agent d'augmentation de flux sanguin comprend du tPA ou de la streptokinase.

13. Combinaison selon l'une quelconque des revendications 10 à 12 pour l'utilisation selon l'une quelconque des revendications 1 à 9, dans laquelle ledit polypeptide est destiné à une administration avant, durant ou après administration dudit agent d'augmentation de flux sanguin afin d'augmenter le flux sanguin.
